# EUROPEAN PATENT SPECIFICATION

(11) **EP 3 857 563 B1**
(45) Date of publication and mention of the grant of the patent: **13.08.2025**
(21) Application number: 19770123.8
(22) Date of filing: 24.09.2019
(51) Int. Cl.: G16H 40/63

(54) **MEDICAL MONITORING SYSTEM**
SYSTEM ZUR MEDIZINISCHEN ÜBERWACHUNG
SYSTÈME DE SURVEILLANCE MÉDICALE

(30) Priority: 24.09.2018 EP 18196214
(43) Date of publication of application: 04.08.2021
(73) Proprietor: Koninklijke Philips N.V., 5656 AG Eindhoven (NL)
(72) Inventor: GREINER, Harald, 5656 AE Eindhoven (NL)
(74) Representative: Philips Intellectual Property & Standards
(86) International application number: PCT/EP2019/075697
(87) International publication number: WO 2020/064722

(56) References cited:
- WO-A1-2015/078710
- WO-A1-2018/019942
- WO-A2-2012/040554
- US-A1- 2003 025 604
- ARNAUD VERDANT ET AL: "Low Power Motion Detection with Low Spatial and Temporal Resolution for CMOS Image Sensor", 2006 INTERNATIONAL WORKSHOP ON COMPUTER ARCHITECTURE FOR MACHINE PERCEPTION AND SENSING, 1 September 2007 (2007-09-01), pages 12 - 17, XP055572237, ISBN: 978-1-4244-0686-9, DOI: 10.1109/CAMP.2007.4350342

## Description

### FIELD OF THE INVENTION

The invention relates to a medical monitoring system for monitoring a subject. The invention further relates to a control method, and a computer program for controlling the medical monitoring system.

### BACKGROUND OF THE INVENTION

US2003025604A1 relates to automatically locally controlling a monitoring device according to user preferences.

WO2012040554A2 relates to systems and methods utilizing video cameras for monitoring patients, caregivers, equipment, and other items within a room in a caregiver setting, such as a hospital, nursing home, treatment center, or the like.

WO2015078710A1 relates to a patient monitor and a corresponding method for monitoring a patient.

WO2018019942A1 relates to a patient monitoring system, method and computer program, wherein the patient monitoring system comprises an output device.

Verdant et al. (2006) Int. Workshop Comp. Arch. Mach. Pers. Sens. 12-17 presents algorithms for low power motion detection, and their possible implementation for low power motion detection with low spatial and temporal resolution for CMOS image sensor.

In the field of medical monitoring, many medical parameters are generally measured like the heart rate, the breathing rate, the blood pressure, the oxygen saturation or other vital signs. Especially in hospitals with often thousands of patients a huge amount of data is thereby generated, which need to be stored, in order to be able to, for instance, decide what exactly happened during certain times where significant events occurred. This can be particularly important in situations in which a patient dies. Storing the huge amount of medical parameter data requires a relatively large data storage capacity. Further, reviewing the stored information might also require a lot of human resources.

### SUMMARY OF THE INVENTION

It is an object of the present invention to provide a medical monitoring system for monitoring a subject, a control method, and a computer program for controlling the medical monitoring system, which allow for a reduced required storage capacity. The invention is defined by the independent claims. The dependent claims define advantageous embodiments.

By modifying the temporary resolution of storing the measured medical parameter in the storing unit depending on the identification of a user (caregiver), the amount of data stored at a certain time can be adapted to whether a certain user (caregiver or medical practitioner) is present or not. The medical monitoring system controller is configured to control the medical monitoring system such that the temporal resolution of storing the medical parameter is increased, if a user has been identified. It is also possible that the medical monitoring system controller is configured to compare, if a user has been identified, the identified user with a list of users, thereby generating a comparison result, and to control the medical monitoring system such that the rate of the temporal resolution of storing the measured medical parameter in the storing unit depends on the comparison result, wherein the temporal resolution of storing the measured medical parameter can be increased, if the comparison result indicates that the identified user is on the list of users. It is therefore not required to store the medical parameter measured over time with a relatively high temporal resolution all the time, but this temporal resolution rate of storing might generally be relatively low. The temporal resolution rate of storing is increased if a user has been identified, especially if the identified user is on a predefined list of users. This allows for a significant reduction of the required storage capacity.

The identification unit is preferentially configured to authenticate a user for identifying the user. Thus, preferentially the identification is an authentication of a user, and in a preferred embodiment the identification unit could also be regarded as being an authentication unit. In particular, the controller is configured to control the medical monitoring system such that the temporal resolution of storing the measured medical parameter in the storing unit depends on the authentication of a user. The term *"identification"* can hence refer to a simple identification without authentication, but it preferentially refers to an authentication which inherently includes a simple identification. For instance, a simple identification can be regarded as being the ability to identify uniquely a user, for example, by using a user ID, i.e. the identification unit may just request the user to input a user ID for allowing the identification unit to uniquely identify the user. However, if the identification unit is configured to authenticate the user, in this example the identification unit also requests a password and checks whether the password is correct for the input user ID, in order to authenticate the user. The authentication can hence allow the identification unit to prove that a user is genuinely who that person claims to be and/or to prove that a user is really the person who the identification unit initially believes he/she is. Although in the above example the identification unit is configured to use a user ID and a password for authenticating and thereby also identifying the person, the identification unit can of course also be configured to use other known authentication means.

The medical monitoring system controller can comprise inputs and outputs for communicating with the different components of the medical monitoring system. For instance, it can comprise an input for receiving a signal from the identification unit, which indicates that a user has been identified, particularly authenticated. It might be possible that the medical monitoring system controller is in a same casing together with the medical parameter measuring unit and/or the storing unit, wherein the identification unit can be in another casing. For example, the identification unit can be a separate device, which can be located next to a casing comprising the medical parameter measuring unit, the storing unit and the medical monitoring system controller, wherein the medical monitoring system controller can comprise an input to which the identification unit can be connected, possibly via a connector at the outside of the casing, in order to allow the identification unit to provide a corresponding signal to the medical monitoring system controller. However, the different components of the medical monitoring system can also be distributed in another way. For example, they can all be included in a single casing and form a single device, they can be distributed among several rooms in a hospital, et cetera.

In an embodiment, the identification unit, the storing unit and the medical monitoring system controller are included in a first device, i.e., for instance, in a first casing, wherein the medical parameter measuring unit is or part of a second device being spatially separated from the first device. In particular, the first device and the second device can be located adjacent to each other, wherein the first device and the second device can be adapted to allow for a data connection between these two devices, in order to allow for a control of the medical monitoring system comprising the two devices such that the temporal resolution of storing the measured medical parameter in the storing unit depends on the identification of a user.

The medical parameter measuring unit can be adapted to measure one or several medical parameters. If a current temporal resolution of measuring one or several medical parameters to be stored is too low such that the one or more medical parameters cannot be stored with a required increased temporal resolution, the medical monitoring system controller controls the medical parameter measuring unit such that the temporal resolution rate of measuring the one or several medical parameters is increased accordingly.

This might have an advantage that the earlier defined (by a caregiver upon patient's admittance, for example) rate of the medical parameter acquisition might be too low to for the currently identified clinical event (requiring highly skilled qualified practitioner's presence) imposing an increased data rate storage. In this case the controller would enable the measuring system to switch to a higher medical data rate acquisition mode without practitioner's intervention. This would save time, such that the medical practitioner would be focused on providing his care to the patient rather than being busy with setting up medical equipment.

In an embodiment, the medical monitoring system controller is configured to determine whether the measured medical parameter fulfils a predefined condition and to control the medical monitoring system such that the temporal resolution of storing the measured medical parameter in the storing unit depends on the fulfilment of the predefined condition. In particular, the medical parameter measuring unit is configured to measure several medical parameters over time, thereby generating several medical parameter waveforms, wherein the medical monitoring system controller is configured to combine the several medical parameter waveforms for generating a combination result, to determine whether the combination result fulfils a predefined condition and to control the medical monitoring system such that the temporal resolution of storing the measured medical parameters in the storing unit depends on the fulfilment of the predefined condition. For instance, if the predefined condition is fulfilled, the temporal resolution rate of storing the measured medical parameter can be increased. In this embodiment the temporal resolution rate of storing the measured medical parameters does not only depend on the identification of a user, but also on the measured medical parameters. This can further ensure that, although generally the measured medical parameter is stored with a relatively low temporal resolution, at medically relevant events, the temporal resolution of storing the measured medical parameter is nevertheless relatively high.

In an embodiment, the medical monitoring system controller is configured to control the medical monitoring system such that the temporal resolution of measuring the medical parameter depends on the identification of a user. In particular, the medical monitoring system controller can be configured to control the medical monitoring system such that the temporal resolution of measuring the measured medical parameter is increased, if a user has been identified. In an embodiment the medical parameter measuring unit comprises a camera for acquiring images of the subject over time, wherein the medical parameter measuring unit is configured to determine the medical parameter based on the acquired images, wherein the medical monitoring system controller is configured to control the medical monitoring system such that the frequency of acquiring the images and hence the temporal resolution of measuring the medical parameter depends on the identification of a user. The camera can be a camera, which is adapted to acquire single images and/or it can be a camera, which acquires a video, i.e. several images with a relatively low temporal distance. The medical parameter measuring unit can be configured to determine, for instance, the heart rate and/or the breathing rate by sensing changes in skin color and/or body movements. The camera can be configured to be operated in the infrared wavelength range and/or in the visible wavelength range. If the medical parameter is determined based on the acquired image, the frequency of acquiring the images can depend on the identification of the user and optionally also on whether the medical parameter fulfils a predefined condition. For instance, the frequency of acquiring the images, i.e. in the case of a video, the frame rate, can be increased, if a user has been identified and optionally if the measured medical parameter fulfils a predefined condition, in order to allow for an increased temporal resolution of storing the medical parameter which is determined based on the acquired images.

It is preferred that the medical monitoring system further comprises a medical report generation unit configured to generate a medical report including the measured medical parameter, wherein the storing unit is configured to store the medical parameter by storing the medical report, wherein the medical monitoring system controller is configured to control the medical monitoring system such that the temporal resolution of the medical parameter included in the medical report depends on the identification of a user.

The medical parameter measuring unit is preferentially configured to measure vital signs as medical parameters. In particular, the medical parameter measuring unit is configured to measure at least one medical parameter of a list consisting of the heart rate, the blood pressure, the oxygen saturation and the breathing rate.

It shall be understood that the medical monitoring system, the control method, and the computer program have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

Fig. 1 shows schematically and exemplarily an embodiment of a medical monitoring system for monitoring a subject,
Fig. 2 shows schematically and exemplarily a further embodiment of a medical monitoring system for monitoring a subject,
Fig. 3 shows schematically and exemplarily an even further embodiment of a medical monitoring system for monitoring a subject.
Fig. 4 shows a flowchart exemplarily illustrating an embodiment of a control method for controlling a medical monitoring system for monitoring a subject.

### DETAILED DESCRIPTION OF EMBODIMENTS

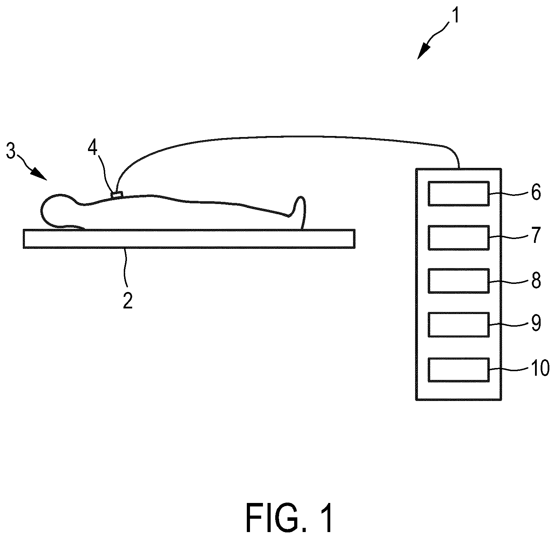

Fig. 1 shows schematically and exemplarily an embodiment of a medical monitoring system for monitoring a subject. In this embodiment the subject is a patient 3 lying in a bed 2, wherein the medical monitoring system 1 comprises a medical parameter measuring unit 4, 6 configured to measure a medical parameter of the subject over time. In the context of the present application, it shall be understood that the medical parameter measuring unit can be a medical sensor assembly arranged to acquire vital signs of a patient, wherefrom various physiological medical parameters can be derived.

The medical parameter measuring unit can include a detector or sensor 4 for generating a detection signal depending on a vital sign being, in this embodiment, the medical parameter, wherein the detector 4 might be an electrical detector, an optical detector, a magnetic detector, an ultrasound sensor or another type of detector. The detector 4 can comprise, for instance, an electrode like an electrocardiography (ECG) electrode, an optical photoplethysmography (PPG) detector and/or an ultrasound sensor array. The medical parameter measuring unit further comprises a processing unit 6 for processing the detection signal received from the detector 4 and for determining a value being indicative of the measured medical parameter. The medical parameter measuring unit can be adapted to derive one or several medical parameters from the acquired detection signals like the heart rate, the breathing rate, the blood pressure, the oxygen saturation, et cetera.

It should be noted that in Fig. 1 the position of the detector 4 on the patient 3 might not be the real position in a real situation, i.e. the position of the detector 4 of course depends on the kind of detector used for measuring the medical parameter. For instance, if the detector comprises an ECG electrode, it might be located on the chest of the patient 3, and, if the detector 4 is a PPG detector, it might be positioned at a finger of the patient 3.

The medical monitoring system 1 further comprises an identification unit 7 configured to authenticate a user. The user to be authenticated is a physician or a caregiver like a nurse. The identification unit 7 can be configured to use known authentication means like the use of passwords, RFID chips, ID cards, fingerprints, face recognition, et cetera.

The medical monitoring system 1 also comprises a storing unit 8 configured to store the measured medical parameter, a display 9 configured to display the measured medical parameter and a medical monitoring system controller configured to control the medical monitoring system 1. The storing unit 8 can be either physically located at the patient's side or be a part of a centralized patient monitoring and administration system of the hospital facility located away from the patient's side (for example, a virtual cloud etc.)

The measured medical parameter, often in a shape of a temporally varying waveform, is visualized on a display of the medical monitoring system. The values of these medical parameter readings are coupled to a medical alarm monitoring unit, functions of which can be also performed by the processing unit 6. The medical alarm monitoring unit analyses acquired medical parameter values in real time and is arranged to cause a warning to the user (caregiver) once the patient enters a clinically critical state. Commonly the acquired medical parameter readings, which are used for monitoring patient's status in real time, are stored in the storing unit 8, as a part of electronic medical records system for example, at a predefined temporal resolution rate which is one averaged number (medical parameter) per minute or 5 minutes. Historically the reduced amount of stored data compared to the real-time parameter monitoring had to do with technical delays associated with physical data storage and amount memory required to keep and maintain the acquired data. Further, in the past clinical practitioners used to oversee less patients and had more time to oversee the patient's progress without looking up electronic medical records.

Nowadays healthcare environment is evolving. There are fewer medical practitioners per one patient, thus less time for the caregiver to be on top of the patient's status. Moreover, the medical environment becomes more digitized: more waveforms are visualized on the display. All these starts leading to an increased workload of the medical stuff and an increased number of burnouts in the medical practitioners' community. Therefore, even if the current technology could permit storing real-time waveforms of each patient, it is highly inefficient not only from sustainability point of view (storing and keeping data requires energy and material resources), but from the clinical need point of view.

The present invention introduces the medical monitoring system controller 10 with a new function, which enables it controlling the medical monitoring system 1 such that the temporal resolution of storing the measured medical parameter in the storing unit 8 depends on the authentication of a user. In other words, the medical monitoring system controller is configured to control the medical monitoring system to increase a rate of its temporal resolution storing of the measured medical parameter in the storing unit based on an event of the identification of the user.

Moreover, in an embodiment the medical monitoring system controller is configured to compare, if a user has been authenticated, the authenticated user with a list of users, thereby generating a comparison result, and to control the medical monitoring system 1 such that the temporal resolution of storing the measured medical parameter is increased if the comparison result indicates that the authenticated user is on the list of users. Such a comparison can be achieved by coupling the medical monitoring controller to one of the data bases of the medical facility, wherein the data based might comprise staff information including such a list of users.

One of the advantages is that, if a medical alarm is triggered by the medical alarm monitoring unit a medical specialist will be prompted to attend the patient. Once the identification unit 7 identifies such a user (for example, based on reviewing the list of users and whether said medical specialist belongs to the list) the controller will control the storing unit 8 to store the measured medical parameter at a different temporal resolution rate, being higher than the temporal resolution rate prior to the user's identification.

For example, an acquisition sample rate of a sensor providing signals for the heart rate derivation is about 250 Hz, while an acquisition sample rate of the PPG sensor is about 125 Hz. At these temporal resolutions a heart rate and SpO₂ waveforms are visualized by the medical monitoring system in this example. A regular temporal resolution of these waveforms, at which they are stored in the storage unit for further reporting purposes, for example, is one averaged number per minute or 5 minutes. Once the user authentication event occurs the temporal resolution is increased to, for instance, one averaged number per minute or to a higher temporal resolution, to one averaged number per 30 seconds or to a higher resolution, or even to one averaged number per 15 seconds or to a higher resolution. Instead of or in addition to storing an averaged number it is also possible that non-averaged numbers are stored. In this example, preferentially, for each measured medical parameter an averaged number and/or a non-averaged number is stored with the increased temporal resolution, if a user has been authenticated.

This way the medical monitoring system 1 enables an automated optimized recording of the clinically relevant to the patient information, which enables the clinical practitioners reviewing the changes in time of medical parameters at the desired level of details for a given period of time. For example, nowadays general ward (GW) units are reluctant to adapt more monitoring solutions, because in contrast to the intensive care units (ICU) the GW units have less qualified stuff and resources to professionally act on monitored information (as well as manage it). However, it is often the case that patients experience complications or even die because of reduced care in the GW compared to the ICUs.

The present invention provides a solution, which would allow collecting the monitoring information at the GW units in the most optimized way. This would enable the care givers to review in more detail the medical readings of the patient's while a caregiver attends the patient's side in order to identify complicated clinical events, which would require more attention in future.

The medical monitoring system controller 10 can also be configured to determine whether the measured medical parameter fulfils a predefined condition and to control the medical monitoring system 1 such that the temporal resolution of storing the measured medical parameter in the storing unit 8 depends on the fulfillment of the predefined condition. For instance, the medical parameter measuring unit 4, 6 can be configured to measure several medical parameters over time, thereby generating several medical parameter waveforms, wherein the medical monitoring system controller 10 can be configured to combine the several medical parameter waveforms for generating a combination result. The medical monitoring system controller 10 can then be further adapted to determine whether the combination result fulfils a predefined condition and to control the medical monitoring system 1 such that the temporal resolution of storing the measured medical parameters in the storing unit 8 depends on the fulfillment of the predefined condition. In particular, the temporal resolution of storing the measured medical parameters can be increased, if the predefined condition is fulfilled.

The medical monitoring system controller 10 can be configured to control the storing unit 8 to store the measured medical parameter at a first temporal resolution rate and a second temporal resolution rate, being higher than the first temporal resolution rate, while when the medical monitoring system controller determines the fulfilment of the predefined condition it is further arranged to alter the temporal resolution rate to a third temporal resolution rate of storing the measured medical parameter in the storing unit. In an example, the third temporal resolution rate can be larger than the second one.

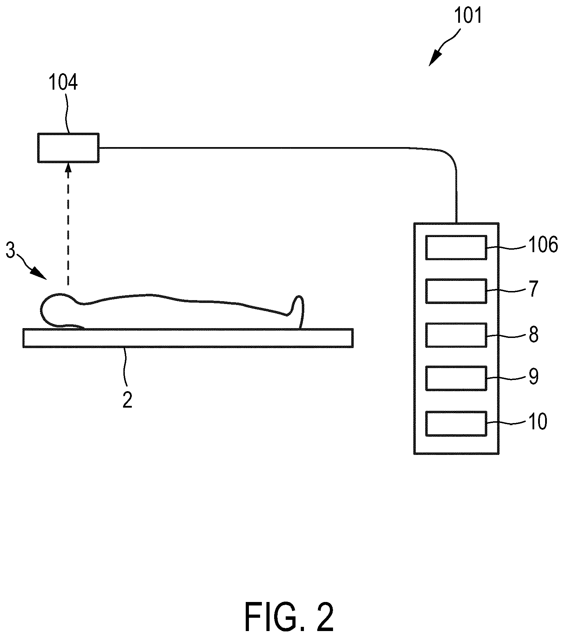

Fig. 2 shows schematically and exemplarily a further embodiment of a medical monitoring system for monitoring a subject. This embodiment of the medical monitoring system is similar to the embodiment described above with reference to Fig. 1, wherein, instead of the medical parameter measuring unit 4, 6, the medical monitoring system 101 shown in Fig. 2 comprises a camera 104 and a processing unit 106, which form the medical parameter measuring unit in this embodiment. The camera 104 is configured to acquire images of the subject over time, particularly a video of the subject 3, wherein the processing unit 106 is configured to determine the medical parameter based on the acquired images. Thus, in this embodiment the medical parameter like the heart rate or the breathing rate can be determined without needing to contact the subject 3. Moreover, in this embodiment the medical monitoring system controller 10 can be configured to control the medical monitoring system 101 such that the frequency of acquiring the images depends on the authentication of the user. For instance, if a user on the user list has been authenticated, the frequency of acquiring the images, i.e. the frame rate if the camera acquires a video, can be increased, in order to allow for an increased resolution of measuring the medical parameter and hence for an increased temporal resolution of storing the medical parameter. The processing unit 106 can be adapted to use the technique provided by the Vital Signs Camera of the company Philips for determining, for instance, the heart rate or the breathing rate based on the acquired images.

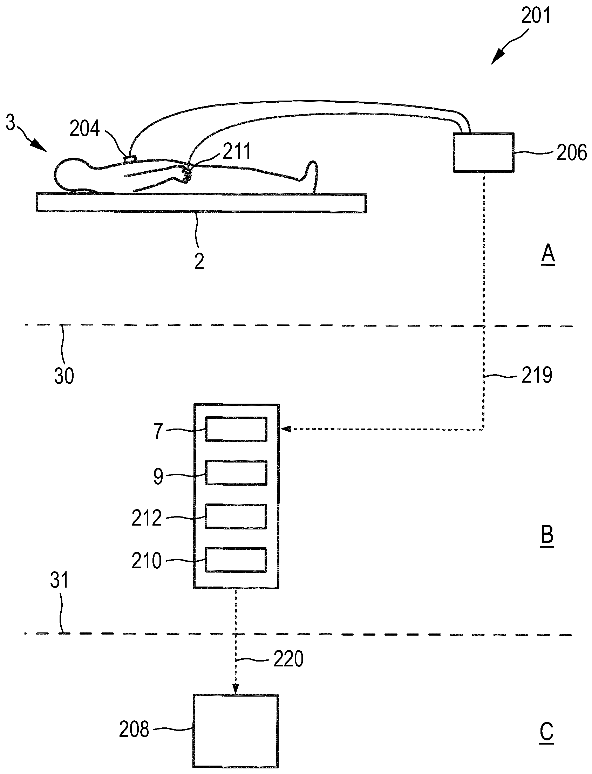

Fig. 3 schematically and exemplarily shows a further embodiment of a medical monitoring system for monitoring a subject. In this embodiment the medical monitoring system 201 comprises a medical parameter measuring unit 204, 206, 211 configured to measure medical parameters of the subject 3 over time in a patient room A. The medical parameter measuring unit comprises a first detector 204 for generating first detection signals being indicative of a first medical parameter of the patient 3 and a second detector 211 for generating a second detection signal being indicative of a second medical parameter of the patient 3. The detection signals are processed by a processing unit 206 for determining respective values of the respective medical parameter. For instance, the processing unit 206 can be configured to determine the heart rate, the respiratory rate, the blood pressures and/or the oxygen saturation based on the received detection signals. The first detector can comprise, for instance, an ECG electrode and the second detector 211 can be, for instance, a PPG detector. The measured medical parameters are transmitted via a data connection 219 to another room B which might be a room where the caregivers like nurses are normally located, if they are not in a patient room like the patient room A. In Fig. 3, the dashed lines 30, 31 illustrate borders like walls between the different rooms.

In the room B a further part of the medical monitoring system 201 is located. This further part comprises the identification unit 7 configured to authenticate a user, one or several displays 9 for showing the measured medical parameters, a medical report generation unit 212 configured to generate a medical report about the patient 3, which includes the measured medical parameters, and a medical monitoring system controller 210 for controlling the medical monitoring system 201. In this embodiment the storing unit 208 is configured to store the medical parameters by storing the medical report and it is located in a further room C, wherein the generated medical report is transmitted to the storing unit 208 via a data connection 220. In this embodiment the storing unit 208 can be regarded as being an electronic patient record database in which the medical reports can be stored.

The medical monitoring system controller 210 is configured to control the medical monitoring system 201 such that the temporal resolution of the medical parameters included in the medical report depends on the authentication of a user. Thus, if a user like a staff member of a hospital is authenticated by the identification unit 7 in the room B, this authentication event can trigger an e-report creation, i.e. a creation of the medical report by the medical report generation unit 212, with an increased temporal resolution, i.e. with a higher sampling rate, of medical parameters which preferentially include vital signs waveforms.

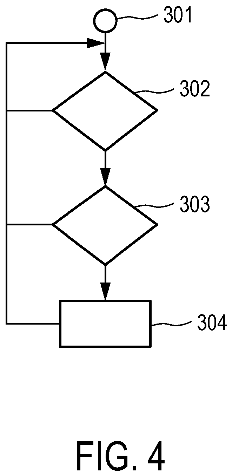

In the following an embodiment of a control method for controlling a medical monitoring system for monitoring a subject will exemplarily be described with reference to a flowchart shown in Fig. 4.

In step 301 the control method is started, wherein this includes starting a continuous measurement of medical parameters of a subject over time by one or several medical parameter measuring units. This measurement of the medical parameters is continuously carried out (at a predefined temporal acquisition rate), while the following steps are performed. In step 302 it is determined whether an identification unit has authenticated a user (a medical practitioner or a caregiver). If this is the case, in step 303 it may be further determined whether the authenticated user is a member of a predefined list of users, wherein, if also this is the case, in step 304 the temporal resolution rate of storing the measured medical parameters is increased. If the temporal resolution of the measurement of the medical parameters is not sufficient for storing the medical parameters with the increased temporal resolution, in step 304 also the temporal resolution of the measurement rate (or an acquisition rate) of the medical parameters is increased.

Thus, in this embodiment not any authentication of a user leads to an increased temporal resolution of storing the medical parameters, but the temporal resolution is only increased if the authenticated user is in the predefined list of users. In another embodiment, step 303 can be omitted such that each authorization of a user leads to an increased temporal resolution of storing the medical parameters.

The patient monitoring systems and the control method described above with reference to Figs. 1 to 4 allow for an altered temporal resolution rate of acquired medical data storage (medical data input-wise) triggered by a predefined event during a patient's stay at a hospital, wherein the predefined event is at least an authentication of a user like a physician or a nurse. Storing all medical parameters obtained in a hospital with a rather deep and high resolution requires a relatively large storage capacity such that it would be advantageous, if the medical parameters measured over time for the often thousands of patients in a hospital could be stored with a lower resolution. However, in clinical settings it is sometimes important to be able to decide what exactly happened in certain situations with possibly extreme outcomes like a death of a patient. The medical monitoring systems and control method described above with reference to Figs. 1 to 4 allow to generally reduce the temporal resolution rate of storing the measured medical parameters, wherein, if a relevant situation occurs as indicated by the authentication of a user, the temporal resolution of storing the measured medical parameters is increased, in order to ensure that in relevant situations the temporal resolution is high enough.

The processing part of the medical parameter measuring unit like the processing unit 6, 106, 206, the identification unit, the storing unit, and the medical monitoring system controller can all be included in a single device which might be regarded as being a patient monitor as illustrated in Figs. 1 and 2. However, it is also possible that, for instance, the storing unit is not part of such a patient monitor, but an external device which is not included in the patient monitor which, in this example, includes the processing unit of the medical parameter measuring unit and optionally the identification unit and the medical monitoring system controller. Thus, the medical parameter to be stored can be made available to an external device which might nevertheless be regarded as being part of the overall patient monitoring system as exemplarily described above with reference to Fig. 3.

Fig. 3 illustrates an ecosystem that triggers the increased temporal resolution of storing the medical parameters and documents in a sensible way, without requiring extensive data communication and storage, wherein the ecosystem can still run fully automatic in the background without any user intervention, except for a user interaction which might be required for the authentication, wherein the high resolution documentation can be provided if required in case of, for instance, legal proceedings.

The medical monitoring system and control method described above with reference to Figs. 1 to 4 allow triggering a detailed data storage process for around a relevant situation which is indicated at least by an authentication of a user. The storage of the detailed medical data, i.e. of the measured medical parameters, by storing them with a higher temporal density than usually required for a medical trend assessment, can be performed by, for instance, a respective patient monitor itself or the detailed medical data can be made available to an external storing unit.

It shall be also understood by the person skilled in the art that the invention can be practiced in a fully automated mode. In an embodiment the medical monitoring system controller, based on an input from the medical parameter measuring unit, which can be also configured to determine whether the measured medical parameter fulfils a predefined condition, and to control storing unit 8, 208 to store the measured medical parameter at an increased temporal resolution rate, wherein said rate is defined by the fulfilment of the predefined condition.

In the above-described embodiments, medical parameters like the heart rate, the breathing rate, the blood pressure, the oxygen saturation, et cetera have been exemplarily mentioned. However, the medical monitoring system can of course also monitor other medical parameters particularly other vital signs. Moreover, although in the above described embodiments the identification unit is adapted to authenticate a user, wherein the medical monitoring system controller is configured to control the medical monitoring system such that the temporal resolution of storing the measured medical parameter in the storing unit depends on the authentication of a user, in another embodiment the identification unit can be adapted to carry out a simple identification, for instance, just by asking for entering a user ID without any password request, wherein the medical monitoring system controller can be configured to control the medical monitoring system such that the temporal resolution of storing the measured medical parameter in the storing unit depends on the simple identification of a user.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the processing of the detection signals for measuring the medical parameter, the authentication, the control of the medical monitoring system, et cetera, performed by one or several units or devices can be performed by any other number of units or devices. These procedures, particularly the control of the medical monitoring system in accordance with the control method carried out by the medical monitoring system controller, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a medical monitoring system comprising a controller for controlling the medical monitoring system. The controller is configured to control the medical monitoring system such that the temporal resolution of storing measured medical parameters like blood pressure, heart rate et cetera in a storing unit depends on the identification of a user who is a physician or nurse, wherein the identification is preferentially an authentication of the user. In particular, the temporal resolution of storing measured medical parameters is increased, if a user has been identified. It is therefore not required to store the medical parameter with a relatively high temporal resolution all the time, but this temporal resolution might generally be relatively low and increased, if a user has been identified. This allows for a significant reduction of the required storage capacity.

## Claims

1. A medical monitoring system (1; 101, 201) comprising:
a medical parameter measuring unit (4, 6; 104, 106; 204, 206, 211) configured to measure a medical parameter of a subject over time,
an identification unit (7) configured to identify a user, wherein the user is a caregiver or a medical practitioner,
a storing unit (8; 208) configured to store the measured medical parameter at a temporal resolution rate, and
a medical monitoring system controller (10; 210) being coupled to the medical parameter measuring unit, the identification unit, and the storing unit, wherein the medical monitoring system controller (10; 210) is configured to control the medical monitoring system (1; 101, 201) to increase the temporal resolution rate of storing the measured medical parameter in the storing unit (8; 208) based on an event of the identification of the user.

2. The medical monitoring system as defined by claim 1, wherein the medical monitoring system controller (10; 210) is configured to compare, when the user is identified, the identified user with a list of users, thereby generating a comparison result, and to control the medical monitoring system (1; 101, 201) to increase the temporal resolution rate of storing the measured medical parameter in the storing unit (8; 208) dependent on the comparison result.

3. The medical monitoring system as defined by claim 2, wherein the medical monitoring system controller (10; 210) is configured to control the medical monitoring system (1; 101, 201) such that the temporal resolution rate of storing the measured medical parameter is increased, if the comparison result indicates that the identified user is on the list of users.

4. The medical monitoring system as defined by any of the preceding claims, wherein the medical monitoring system controller (10; 210) is configured to determine whether the measured medical parameter fulfils a predefined condition and to control the medical monitoring system (1; 101, 201) to further alter the temporal resolution rate to a third temporal resolution rate of storing the measured medical parameter in the storing unit (8; 208) based on the fulfilment of the predefined condition.

5. The medical monitoring system as defined by any of the preceding claims, wherein the medical parameter measuring unit (4, 6; 104, 106; 204, 206, 211) is configured to measure several medical parameters over time, thereby generating several medical parameter waveforms, wherein the medical monitoring system controller (10; 210) is configured to combine the several medical parameter waveforms for generating a combination result, to determine whether the combination result fulfils a predefined condition, and to control the medical monitoring system (1; 101, 201) to further alter the temporal resolution rate of storing the measured medical parameters in the storing unit (8; 208) based on the fulfilment of the predefined condition.

6. The medical monitoring system as defined by any of the preceding claims, wherein the medical monitoring system controller is further coupled to the medical parameter measuring unit and configured to control the medical monitoring system such that the temporal resolution rate of measuring the medical parameter is altered on an event of the identification of a user.

7. The medical monitoring system as defined by claim 6, wherein the medical monitoring system controller is configured to control the medical parameter measuring unit to acquire a medical parameter of the subject over time with a first temporal resolution rate of measuring and a second resolution rate of measuring being higher than the first temporal resolution rate of measuring, wherein said second resolution rate of measuring the measured medical parameter is applicable, when the user is identified.

8. The medical monitoring system as defined by claims 6 or 7, wherein the medical parameter measuring unit (104, 106) comprises a camera (104) for acquiring images of the subject over time, wherein the medical parameter measuring unit (104, 106) is configured to determine the medical parameter based on the acquired images, wherein the medical monitoring system controller is configured to control the medical monitoring system (101) to alter the frequency of acquiring the images based on an event of the identification of the user.

9. The medical monitoring system as defined by any of the preceding claims, wherein the medical parameter measuring unit (4, 6; 104, 106; 204, 206, 211) is configured to measure at least one medical parameter of a list consisting of the heart rate, the blood pressure, the oxygen saturation, and the breathing rate.

10. A control method for controlling a medical monitoring system for monitoring a subject as defined by any of the preceding claims, wherein the medical monitoring system (1; 101, 201) is controlled such that it carries out:
- measuring a medical parameter of the subject over time by the medical parameter measuring unit (4, 6; 104, 106; 204, 206, 211),
- identifying a user by the identification unit (7), wherein the user is a caregiver or a medical practitioner, and
- storing the measured medical parameter by the storing unit (8; 208) at a first temporal resolution rate and a second temporal resolution rate, being higher than the first temporal resolution rate, wherein the second temporal resolution rate is used when the user is identified.

11. A computer program for controlling a medical monitoring system as defined by claims 1-9, the computer program comprising program code means for causing the medical monitoring system (1; 101, 201) to carry out the steps of the control method as defined in claim 10, when the computer program is run on the medical monitoring system controller (10; 210) controlling the medical monitoring system (1; 101, 201).

## Patentansprüche

1. Medizinisches Überwachungssystem (1; 101, 201), umfassend:
eine Messeinheit (4, 6; 104, 106; 204, 206, 211) medizinischer Parameter, die konfiguriert ist, um einen medizinischen Parameter einer Zielperson im Zeitverlauf zu messen,
eine Identifikationseinheit (7), die konfiguriert ist, um einen Benutzer zu identifizieren, wobei der Benutzer eine Pflegekraft oder ein Arzt ist,
eine Speichereinheit (8; 208), die konfiguriert ist, um den gemessenen medizinischen Parameter mit einer zeitlichen Auflösungsrate zu speichern, und
eine Steuereinheit (10; 210) des medizinischen Überwachungssystems, die mit der Messeinheit medizinischer Parameter, der Identifikationseinheit und der Speichereinheit gekoppelt ist, wobei die Steuereinheit (10; 210) des medizinischen Überwachungssystems konfiguriert ist, um das medizinische Überwachungssystem (1; 101, 201) zu steuern, um die zeitliche Auflösungsrate des Speicherns des gemessenen medizinischen Parameters in der Speichereinheit (8; 208) basierend auf einem Ereignis der Identifikation des Benutzers zu erhöhen.

2. Medizinisches Überwachungssystem nach Anspruch 1, wobei die Steuereinheit (10; 210) des medizinischen Überwachungssystems konfiguriert ist, um, wenn der Benutzer identifiziert ist, den identifizierten Benutzer mit einer Liste von Benutzern zu vergleichen und dadurch ein Vergleichsergebnis zu generieren, und um das medizinische Überwachungssystem (1; 101, 201) zu steuern, um die zeitliche Auflösungsrate des Speicherns des gemessenen medizinischen Parameters in der Speichereinheit (8; 208) abhängig von dem Vergleichsergebnis zu erhöhen.

3. Medizinisches Überwachungssystem nach Anspruch 2, wobei die Steuereinheit (10; 210) des medizinischen Überwachungssystems konfiguriert ist, um das medizinische Überwachungssystem (1; 101, 201) derart zu steuern, dass die zeitliche Auflösungsrate des Speicherns des gemessenen medizinischen Parameters erhöht wird, wenn das Vergleichsergebnis angibt, dass der identifizierte Benutzer auf der Benutzerliste steht.

4. Medizinisches Überwachungssystem nach einem der vorstehenden Ansprüche, wobei die Steuereinheit (10; 210) des medizinischen Überwachungssystems konfiguriert ist, um zu bestimmen, ob der gemessene medizinische Parameter eine vordefinierte Bedingung erfüllt, und um das medizinische Überwachungssystem (1; 101, 201) zu steuern, um basierend auf der Erfüllung der vordefinierten Bedingung die zeitliche Auflösungsrate in der Speichereinheit (8; 208) weiter auf eine dritte zeitliche Auflösungsrate des Speicherns des gemessenen medizinischen Parameters zu ändern.

5. Medizinisches Überwachungssystem nach einem der vorstehenden Ansprüche, wobei die Messeinheit (4, 6; 104, 106; 204, 206, 211) medizinischer Parameter konfiguriert ist, um mehrere medizinische Parameter im Zeitverlauf zu messen und dadurch mehrere Wellenformen medizinischer Parameter zu generieren, wobei die Steuereinheit (10; 210) des medizinischen Überwachungssystems konfiguriert ist, um die mehreren Wellenformen medizinischer Parameter zu kombinieren, um ein Kombinationsergebnis zu generieren, um zu bestimmen, ob das Kombinationsergebnis eine vordefinierte Bedingung erfüllt, und um das medizinische Überwachungssystem (1; 101, 201) zu steuern, um die zeitliche Auflösungsrate des Speicherns der gemessenen medizinischen Parameter in der Speichereinheit (8; 208) basierend auf der Erfüllung der vordefinierten Bedingung weiter zu ändern.

6. Medizinisches Überwachungssystem nach einem der vorstehenden Ansprüche, wobei die Steuereinheit des medizinischen Überwachungssystems weiter mit der Messeinheit medizinischer Parameter gekoppelt und konfiguriert ist, um das medizinische Überwachungssystem derart zu steuern, dass die zeitliche Auflösungsrate des Messens des medizinischen Parameters bei der Identifikation eines Benutzers geändert wird.

7. Medizinisches Überwachungssystem nach Anspruch 6, wobei die Steuereinheit des medizinischen Überwachungssystems konfiguriert ist, um die Messeinheit medizinischer Parameter zu steuern, um einen medizinischen Parameter der Zielperson im Zeitverlauf mit einer ersten zeitlichen Messauflösungsrate und einer zweiten Messauflösungsrate zu erfassen, die höher ist als die erste zeitliche Messauflösungsrate, wobei die zweite Messauflösungsrate des gemessenen medizinischen Parameters anwendbar ist, wenn der Benutzer identifiziert ist.

8. Medizinisches Überwachungssystem nach Anspruch 6 oder 7, wobei die Messeinheit (104, 106) medizinischer Parameter eine Kamera (104) zum Erfassen von Bildern der Zielperson im Zeitverlauf umfasst, wobei die Messeinheit (104, 106) medizinischer Parameter konfiguriert ist, um die medizinischen Parameter basierend auf den erfassten Bildern zu bestimmen, wobei die Steuereinheit des medizinischen Überwachungssystems konfiguriert ist, um das medizinische Überwachungssystem (101) zu steuern, um die Frequenz vom Erfassen der Bilder basierend auf einem Ereignis der Identifikation des Benutzers zu ändern.

9. Medizinisches Überwachungssystem nach einem der vorstehenden Ansprüche, wobei die Messeinheit (4, 6; 104, 106; 204, 206, 211) medizinischer Parameter konfiguriert ist, um mindestens einen medizinischen Parameter aus einer Liste bestehend aus der Herzfrequenz, dem Blutdruck, der Sauerstoffsättigung und der Atemfrequenz zu messen.

10. Steuerverfahren zum Steuern eines medizinischen Überwachungssystems zum Überwachen einer Zielperson nach einem der vorstehenden Ansprüche, wobei das medizinische Überwachungssystem (1; 101, 201) derart gesteuert wird, dass es Folgendes ausführt:
- Messen eines medizinischen Parameters der Zielperson im Zeitverlauf durch die Messeinheit (4, 6; 104, 106; 204, 206, 211) medizinischer Parameter,
- Identifizieren eines Benutzers durch die Identifikationseinheit (7), wobei der Benutzer eine Pflegekraft oder ein Arzt ist, und
- Speichern des gemessenen medizinischen Parameters durch die Speichereinheit (8; 208) mit einer ersten zeitlichen Auflösungsrate und einer zweiten zeitlichen Auflösungsrate, die höher ist als die erste zeitliche Auflösungsrate, wobei die zweite zeitliche Auflösungsrate verwendet wird, wenn der Benutzer identifiziert ist.

11. Computerprogramm zum Steuern eines medizinischen Überwachungssystems nach Ansprüchen 1-9, wobei das Computerprogramm Programmcodemittel umfasst, um das medizinische Überwachungssystem (1; 101, 201) zu veranlassen, die Schritte des Steuerverfahrens nach Anspruch 10 auszuführen, wenn das Computerprogramm auf der Steuereinheit (10; 210) des medizinischen Überwachungssystems laufen gelassen wird, die das medizinische Überwachungssystem (1; 101, 201) steuert.

## Revendications

1. Système de surveillance médicale (1 ; 101, 201) comprenant :
une unité de mesure de paramètre médical (4, 6; 104, 106 ; 204, 206, 211) configurée pour mesurer un paramètre médical d'un sujet au fil du temps,
une unité d'identification (7) configurée pour identifier un utilisateur, dans lequel l'utilisateur est un soignant ou un médecin,
une unité de stockage (8; 208) configurée pour stocker le paramètre médical mesuré à un rythme de résolution temporelle, et
un dispositif de commande de système de surveillance médicale (10 ; 210) qui est couplé à l'unité de mesure de paramètre médical, à l'unité d'identification et à l'unité de stockage, dans lequel le dispositif de commande de système de surveillance médicale (10 ; 210) est configuré pour commander le système de surveillance médicale (1; 101, 201) pour augmenter le rythme de résolution temporelle de stockage du paramètre médical mesuré dans l'unité de stockage (8 ; 208) sur la base d'un événement de l'identification de l'utilisateur.

2. Système de surveillance médicale selon la revendication 1, dans lequel le dispositif de commande de système de surveillance médicale (10 ; 210) est configuré pour comparer, lorsque l'utilisateur est identifié, l'utilisateur identifié avec une liste d'utilisateurs, ce qui permet de générer un résultat de comparaison, et pour commander le système de surveillance médicale (1 ; 101, 201) pour augmenter le rythme de résolution temporelle de stockage du paramètre médical mesuré dans l'unité de stockage (8 ; 208) en fonction du résultat de comparaison.

3. Système de surveillance médicale selon la revendication 2, dans lequel le dispositif de commande de système de surveillance médicale (10 ; 210) est configuré pour commander le système de surveillance médicale (1 ; 101, 201) de telle sorte que le rythme de résolution temporelle de stockage du paramètre médical mesuré augmente, si le résultat de comparaison indique que l'utilisateur identifié est sur la liste des utilisateurs.

4. Système de surveillance médicale selon l'une quelconque des revendications précédentes, dans lequel le dispositif de commande de système de surveillance médicale (10 ; 210) est configuré pour déterminer si le paramètre médical mesuré remplit une condition prédéfinie et pour commander le système de surveillance médicale (1 ; 101, 201) pour modifier davantage le rythme de résolution temporelle jusqu'à un troisième rythme de résolution temporelle de stockage du paramètre médical mesuré dans l'unité de stockage (8 ; 208) sur la base de l'exécution de la condition prédéfinie.

5. Système de surveillance médicale selon l'une quelconque des revendications précédentes, dans lequel l'unité de mesure de paramètre médical (4, 6; 104, 106 ; 204, 206, 211) est configurée pour mesurer plusieurs paramètres médicaux au fil du temps, ce qui permet de générer de multiples formes d'onde de paramètres médicaux, dans lequel le dispositif de commande de système de surveillance médicale (10 ; 210) est configuré pour combiner les multiples formes d'onde de paramètres médicaux pour générer un résultat de combinaison, pour déterminer si le résultat de combinaison remplit une condition prédéfinie, et pour commander le système de surveillance médicale (1; 101, 201) pour modifier davantage le rythme de résolution temporelle de stockage des paramètres médicaux mesurés dans l'unité de stockage (8 ; 208) sur la base de l'exécution de la condition prédéfinie.

6. Système de surveillance médicale selon l'une quelconque des revendications précédentes, dans lequel le dispositif de commande de système de surveillance médicale est en outre couplé à l'unité de mesure de paramètre médical et configuré pour commander le système de surveillance médicale de telle sorte que le rythme de résolution temporelle de mesure du paramètre médical soit modifié lors d'un événement de l'identification d'un utilisateur.

7. Système de surveillance médicale selon la revendication 6, dans lequel le dispositif de commande de système de surveillance médicale est configuré pour commander l'unité de mesure de paramètre médical pour acquérir un paramètre médical du sujet au fil du temps avec un premier rythme de résolution temporelle de mesure et un deuxième rythme de résolution de mesure qui est plus élevé que le premier rythme de résolution temporelle de mesure, dans lequel ledit deuxième rythme de résolution de mesure du paramètre médical mesuré est applicable lorsque l'utilisateur est identifié.

8. Système de surveillance médicale selon les revendications 6 ou 7, dans lequel l'unité de mesure de paramètre médical (104, 106) comprend une caméra (104) pour acquérir des images du sujet au fil du temps, dans lequel l'unité de mesure de paramètre médical (104, 106) est configurée pour déterminer le paramètre médical sur la base des images acquises, dans lequel le dispositif de commande de système de surveillance médicale est configuré pour commander le système de surveillance médicale (101) pour modifier la fréquence d'acquisition des images sur la base d'un événement de l'identification de l'utilisateur.

9. Système de surveillance médicale selon l'une quelconque des revendications précédentes, dans lequel l'unité de mesure de paramètre médical (4, 6 ; 104, 106 ; 204, 206, 211) est configurée pour mesurer au moins un paramètre médical d'une liste composée de la fréquence cardiaque, de la pression artérielle, de la saturation en oxygène et du rythme respiratoire.

10. Procédé de commande pour commander un système de surveillance médicale pour surveiller un sujet selon l'une quelconque des revendications précédentes, dans lequel le système de surveillance médicale (1; 101, 201) est commandé de telle sorte qu'il effectue :
- la mesure d'un paramètre médical du sujet au fil du temps au moyen de l'unité de mesure de paramètre médical (4, 6 ; 104, 106; 204, 206, 211),
- l'identification d'un utilisateur au moyen de l'unité d'identification (7), dans lequel l'utilisateur est un soignant ou un médecin, et
- le stockage du paramètre médical mesuré au moyen de l'unité de stockage (8 ; 208) à un premier rythme de résolution temporelle et à un deuxième rythme de résolution temporelle, qui est plus élevé que le premier rythme de résolution temporelle, dans lequel le deuxième rythme de résolution temporelle est utilisé lorsque l'utilisateur est identifié.

11. Programme informatique pour commander un système de surveillance médicale selon les revendications 1-9, le programme informatique comprenant des moyens de code de programme pour amener le système de surveillance médicale (1; 101, 201) à effectuer les étapes du procédé de commande selon la revendication 10, lorsque le programme informatique est exécuté sur le dispositif de commande de système de surveillance médicale (10 ; 210) commandant le système de surveillance médicale (1 ; 101, 201).
